# EUROPEAN PATENT SPECIFICATION

(11) **EP 3 040 310 B1**
(45) Date of publication and mention of the grant of the patent: **01.03.2017**
(21) Application number: 15150057.6
(22) Date of filing: 05.01.2015
(51) Int. Cl.: C01G 43/00, A61K 49/04, C08J 3/07, C01B 19/00, A61K 51/12, A61K 41/00

(54) **Preparation method of radiation sensitive copolymer carrier for coating radiated nanoparticles and chemotherapy drugs**
Verfahren zur Herstellung eines strahlungsempfindlichen Copolymerträgers zur Beschichtung von bestrahlten Nanopartikeln und Chemotherapeutika
Procédé de fabrication d'un support co-polymère sensible au rayonnement pour l'enrobage de nanoparticules et des médicaments de chimiothérapie irradiées

(43) Date of publication of application: 06.07.2016
(73) Proprietor: Institute of Nuclear Energy Research, Atomic Energy Council, Executive Yuan, Taoyuan 325 (TW)
(72) Inventor: Lin, Wuu-Jyh, 325 Taoyuan (TW); Duh, Ting-Shien, 325 Taoyuan (TW); Li, Wei-Ming, 325 Taoyuan (TW); Li, Ming-Hsin, 325 Taoyuan (TW)
(74) Representative: Lermer, Christoph

(56) References cited:
- US-A1- 2011 092 420
- ZHONGWEI GU: "Development of a reduction-sensitive diselenide-conjugated oligoethylenimine nanoparticulate system as a gene carrier", INTERNATIONAL JOURNAL OF NANOMEDICINE, 1 July 2012 (2012-07-01), page 3991, XP55198194, DOI: 10.2147/IJN.S32961
- NING MA ET AL: "Dual Redox Responsive Assemblies Formed from Diselenide Block Copolymers", JOURNAL OF THE AMERICAN CHEMICAL SOCIETY, vol. 132, no. 2, 20 January 2010 (2010-01-20), pages 442-443, XP55198084, ISSN: 0002-7863, DOI: 10.1021/ja908124g
- Ning Ma ET AL: "Supporting Information Dual Redox Responsive Assemblies Formed From Diselenide Block Copolymers", Journal of American Chemical Society, 18 December 2009 (2009-12-18), pages s1-s6, XP55198087, Retrieved from the Internet: URL:http://pubs.acs.org/doi/suppl/10.1021/ ja908124g/suppl_file/ja908124g_si_001.pdf [retrieved on 2015-06-24]
- NING MA ET AL: "Radiation-Sensitive Diselenide Block Co-polymer Micellar Aggregates: Toward the Combination of Radiotherapy and Chemotherapy", LANGMUIR, vol. 27, no. 10, 17 May 2011 (2011-05-17), pages 5874-5878, XP55197873, ISSN: 0743-7463, DOI: 10.1021/la2009682

## Description

### BACKGROUND OF THE INVENTION

### 1. Field of the Invention

The field of the invention relates to a method of preparing the radiation-sensitive copolymer carrier for coating radiated nanoparticles and/or chemotherapy drugs used in proton therapy.

### 2. Description of the Related Art

Cancer, also known as malignant tumor, has been the first place of top ten causes of death for a long time; especially lung cancer and liver cancer are the most common types of cancer. Cancer occurs due to human cell diseases, and is treated in common clinical treatment of surgery, radiation, therapy, chemotherapy, and targeted therapy so far. Different treatment effects toward focus of infection of cancer vary from indications to assorted treatments.

The basic principle of radiation therapy is utilizing radioactivity to block the double helical chains in DNA (deoxyribonucleic acid) of cancer cell nucleus for the purpose of killing the cancer cells or inhibiting their growth. Traditional photon therapy that produces gamma radiation or X-radiation through human body, while the energy decaying exponentially relative to the increasing depth into tissue, many other normal tissue would be influenced by the radioactivity before cancer cells being destroyed. The feature of proton therapy is that the energy increasing with distance and slowing down at the range end, namely the location of targeted tumor, to release the maximum energy in an instant, forming a Bragg peak for providing effective treatment to cancer cells with high doses of chemotherapy drugs, and being almost no harm to healthy tissue. For the sake of proton therapy having the feature of spread-out Bragg peak (SOBP), the risk of damaging normal tissue during treatment can be reduced along with a minimum side effect relatively.

The physical characteristics of proton and X-radiation are different. X-radiation can treat tumor located in deep tissue for powerful penetration that accompanies defective effect of leaving high doses at forward tissue before reaching the tumor and damaging adjacent normal tissue with considerable residual doses after penetrating the tumor. Proton releases little energy during through tissue to reach the tumor, but discharges large energy in the tumor after reaching preferred depth of the tumor, the feature is called Bragg peak, without leaving any energy on normal tissue after penetrating the tumor. Because a single Bragg peak is not wide enough, it is necessary to combine several Bragg peaks to expand it to tumor size for enhancing proton therapeutic effect. Proton therapy is currently the most advanced tumor radiation therapy technology in the world for little damage to normal cells around the focus of infection and less side effects relatively, and is expected to be universalized in the future.

Traditional radiation therapy utilizes X-radiation to position and treat tumor, but is unable to accurately control the position and the dose for the tumor to avoid normal tissue between body surface and the tumor from receiving the dose and being damaged. A therapeutic method with accurate positioning and dose is in demand for cancer treatment. Zhongwei Gu et al. (International Journal of Nanomedicine 2012:7 3991-4006) discloses a reduction-sensitive diselenide-conjugated oligoethylenimine nanoparticulate system as a gene carrier.

US patent publication No.2007/0031337 A1 discloses a method of proton tomography utilizing good combination characteristic of gold nanoparticle with antibody to attract antigen in the cancer cells to achieve better treatment positioning and chemotherapy drug dose control. Utilizing proton computer tomography (PCT) system for cancer treatment can be expected to become a trend in the future. However, the cited reference, US patent publication No.2007/0031337 A1, didn't disclose the preparation of a carrier that can be utilized for coating radiated nonoparticles and/or chemotherapy drugs.

### SUMMARY OF THE INVENTION

The primary object of the present invention is to provide a preparation method of radiation-sensitive copolymer carrier for coating nanoparticles and/or chemotherapy drugs. The method includes utilizing polymer of diselenide and 3-aminopropylpoly (ethylene gylycol) to react with each other to obtain diselenide block copolymer as a nano pharmaceutical carrier having hydrophilicity and hydrophobicity for forming a nanosphere by self-assembling in emulsification.
1,2-Distearoyl-sn-Glycero-3-Phosphoethanolamine-PEG-biomarker (hereinafter referred to as DSPE-PEG-biomarker) is another polymer having hydrophilicity and hydrophobicity alike that can be added into the diselenide block copolymer during the emulsification. A stable structure of nanosphere is formed during self-assembling of the polymers, wherein hydrophobic groups of the polymers gathering is arranged by organic solvents, and hydrophilic groups of the polymers are exposed to external solution. The nanosphere produces water-repellent effect due to inner hydrophobicity after volatilization of the organic solvents.

The water-repellent effect makes the nanosphere be indestructible by blocking water molecules into the nanosphere and stable in solution with the outer hydrophilic groups of the nanosphere. The nanosphere produced by radiation-sensitive diselenide block copolymer of the present invention is stable in aqueous solution and preferable to be a potential nuclear pharmaceutical carrier for controlling collapse speed of the carrier by specific radiation.

### BRIEF DESCRIPTION OF THE DRAWINGS

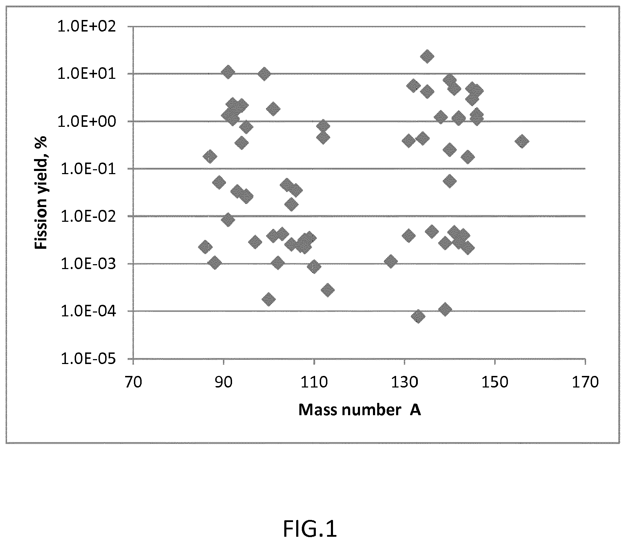

Fig.1 is a chart of changes in distribution of radionuclide fission yield and mass number A.

### DETAILED DESCRIPTION OF THE PREFERRED

### EMBODIMENTS

One preferred embodiment of preparation method of radiation-sensitive copolymer carrier for coating radiated nanoparticles and/or chemotherapy drugs of the present invention, comprising the steps of:
step11: dissolving 2g of 0.05 mole solid sodium hydroxide (NaOH) into 25ml of water, and adding 3.95g of 50 mole selenium powder and 100mg of C₁₉H₄₂BrN (cetyltrimethylammonium bromide, CTAB) to obtain a selenium solution;
step12: dissolving 0.25g of 6.6mole sodium borohydride (NaBH₄) and 0.2g of solid sodium hydroxide into 5ml of water in ice bath to obtain a sodium borohydride solution, and instilling the selenium solution obtained in step11 into the sodium borohydride solution to react under room temperature for about an hour, then reacting at about 90°C for about half an hour to complete reaction and to obtain a red-brown alkaline solution of sodium selenide (Na₂Se₂);
step13: dissolving 2-Dodecen-1-yl-succinic anhydride into (CH₂)₄O (Tetrahydrofuran,THF) and adding into the alkaline solution of Na₂Se₂ to react for about 12 hours, separating impurities by column chromatography and utilizing anhydrous Na₂SO₄ or MgSO₄ to remove water to obtain diselenide after high temperature drying;
step14: dissolving the diselenide obtained in step13 into tetrahydrofuran with polyethylene glycol polymer having amino group, and adding 1-Ethyl-3 (-3-dimethylaminopropyl) carbodiimide (EDC) or N-Hydroxysuccinimide (NHS) to react for about 12 hours, separating impurities by column chromatography to obtain diselenide block copolymer after high temperature drying.

The method of binding diselenide block copolymer with DSPE-PEG-biomarker to form a nanosphere as carrier for coating radiated nanoparticles and/or chemotherapy drug has three types which are described as follows, respectively.
1. The method of coating radiated nanoparticles comprises: dissolving 10mg of diselenide block copolymer as the main ingredient of the carrier and 2mg of DSPE-PEG-biomarker as stabilizer for forming a nanosphere structure of the carrier with 5ml of ultrapure water; adding 1ml of dichloromethane organic solvent having 4mg of oil phase radiated nanoparticles dissolved, wherein the proportion of diselenide block copolymer, DSPE-PEG-biomarker, and oil phase radiated nanoparticles is 5:1:2; completing emulsification with sonication in ice bath; and heating to 60°C to remove the dichloromethane organic solvent to obtain radiation-sensitive nanoparticles that carry radiated nanoparticles (RNPs-Radiation-Sensitive nanoparticles) in size of about 100 nanometers.
2. The method of coating chemotherapy drug comprises: dissolving 10mg of diselenide block copolymer and 2mg of DSPE-PEG-biomarker with 5ml of ultrapure water; adding 1ml of dichloromethane organic solvent having 4mg of doxorubicine dissolved, wherein the proportion of diselenide block copolymer, DSPE-PEG-biomarker, and doxorubicin is 5:1:2; completing emulsification with sonication in ice bath; and heating to 60°C to remove the dichloromethane organic solvent to obtain radiation-sensitive nanoparticles carrying doxorubicin (DOX-Radiation-Sensitive nanoparticles) in size of about 100 nanometers.
3. The method of coating radiated nanoparticles and chemotherapy drug comprises: dissolving 10mg of diselenide block copolymer and 2mg of DSPE-PEG-biomarker with 5ml of ultrapure water; adding 1ml of dichloromethane organic solvent having 2mg of oil phase radiated nanoparticles and 2mg of doxorubicine dissolved, wherein the proportion of diselenide block copolymer, DSPE-PEG-biomarker, oil phase radiated nanoparticles, and doxorubicin is 5:1:1:1; completing emulsification with sonication in ice bath; and heating to 60°C to remove the dichloromethane organic solvent to obtain radiation-sensitive nanoparticles carrying radiated nanoparticles and doxorubicin (RNPs/DOX-Radiation-Sensitive nanoparticles) in size of about 120 nanometers.

Proton therapy that utilizes chemotherapy drugs made by the radiation-sensitive copolymer carrier of the present invention has decreasing proton energy in proportion with penetrated depth while the high-energy protons are hitting into the human body. The protons energy may decrease to one-third or one-fourth of primary energy while the protons are reaching to the cancer according to the penetration depth and incident energy of the protons. The incident protons having energy at 10-1000MeV will cause fission reaction of uranium-238 with certain probability while impacting the uranium-238 distributed on the cancer. FIG.1 shows the fission reaction while protons are impacting uranium-238, wherein the fission yields varies with the mass distribution. These fission products are usually unstable nuclides and subject to continuing decay reactions. Table 1 lists nuclide names of fission products having higher incidence energy and related data of decay reaction that occurred after nuclear fission following proton (the incident energy of high energy proton is 10∼250MeV) striking uranium-238. These fission products will release high energy electrons during decaying process that is to be applied to destroy cancer cells of tumor for enhancing treatment effect after patients finishing their proton therapy.

**TABLE 1**

| | **yield, %** | **half life** | **decay mode** | **decay energy (keV) (intensity%)** |
|---|---|---|---|---|
| **¹³⁵Te** | 23.0 | 19.0 s | %Beta-=100 | Beta-: 5960(50%),5356(25%),5090(%16) |
| **⁹¹Sr** | 10.8 | 9.63 h | %Beta-=100 | Beta-: 2707(29%),1402(25%),1127(%34.7) |
| **⁹⁹Mo** | 9.9 | 65.94 h | %Beta-=100 | Beta-: 1214(82.4%),437(16.4%) |
| **¹⁴⁰La** | 7.3 | 1.6781 d | %Beta-=100 | Beta-: 1679(19.2%),1246(5.7%),1240(%10.9) |
| **¹³²Te** | 5.5 | 3.204 d | %Beta-=100 | Beta-: 215(100%) |
| **¹⁴⁵La** | 4.8 | 24.8 s | %Beta-=100 | Beta-: 4110(15%),4040(19%),3992(%15) |
| **¹⁴¹Ba** | 4.8 | 18.27 m | %Beta-=100 | Beta-: 3023(7%),2746(18%),2565(%25) |
| **¹⁴⁶La** | 4.3 | 6.27 s | %Beta-=100 | Beta-: 6530(18%),6272(28%),5603(%5.5) |
| **¹³⁵I** | 4.2 | 6.57 h | %Beta-=100 | Beta-: 1388(23.8%),1083(8%),970(%21.9) |

It is obvious from the content mentioned above that the preparation method of radiation-sensitive copolymer carrier for coating radiated nanoparticles and/or chemotherapy drugs of the present invention indeed improves the effect of chemotherapy drugs and the accuracy of drug positioning for the treatment.

The foregoing invention has been described in detail by way of illustration and example for purposes of clarity and understanding. It will be apparent to those of ordinary skill in the art that variations, changes, modifications and alterations may be applied to the compositions and/or methods described herein without departing from the true spirit, concept and scope of the invention.

## Claims

1. A preparation method of radiation-sensitive copolymer carrier for coating radiated nanoparticles and/or chemotherapy drugs, comprising:
dissolving solid sodium hydroxide into water and adding selenium powder and cetyltrimethylammonium bromide to obtain a selenium solution;
dissolving sodium borohydride and another solid sodium hydroxide into water in ice bath to obtain a sodium borohydride solution, and instilling the selenium solution into the sodium borohydride solution for reacting under room temperature for about an hour, then reacting at about 90°C for about half an hour to complete the reaction and to obtain a alkaline solution of sodium selenide;
dissolving 2-Dodecen-1-yl-succinic anhydride into tetrahydrofuran and adding into the alkaline solution of sodium selenide to react for about 12 hours, separating impurities by column chromatography and utilizing anhydrous Na₂SO₄ or MgSO₄ to remove water to obtain diselenide after high temperature drying;
dissolving the diselenide into tetrahydrofuran with polyethylene glycol polymer having amino group, and adding 1-Ethyl-3(-3-dimethylaminopropyl)carbodiimide or N-Hydroxysuccinimide to react for about 12 hours, separating impurities by column chromatography to obtain diselenide block copolymer after high temperature drying.

2. The method of claim 1, wherein the diselenide block copolymer is used for coating radiated nanoparticles comprises: dissolving diselenide block copolymer and DSPE-PEG-biomarker with ultrapure water; adding dichloromethane organic solvent having oil phase radiated nanoparticles dissolved, wherein the proportion of the diselenide block copolymer, the DSPE-PEG-biomarker, and the oil phase radiated nanoparticles is 5:1:2; completing emulsification with sonication in ice bath; and heating to 60°C to remove the dichloromethane organic solvent to obtain radiation-sensitive nanoparticles carrying the radiated nanoparticles (RNPs-Radiation-Sensitive nanoparticles).

3. The method of claim 1, wherein the diselenide block copolymer is used for coating chemotherapy drugs comprises: dissolving diselenide block copolymer and DSPE-PEG-biomarker with ultrapure water; adding dichloromethane organic solvent having doxorubicine dissolved, wherein the proportion of diselenide block copolymer, the DSPE-PEG-biomarker, and the doxorubicin is 5:1:2; completing emulsification with sonication in ice bath; and heating to 60°C to remove the dichloromethane organic solvent to obtain radiation-sensitive nanoparticles carrying the doxorubicin (DOX-Radiation-Sensitive nanoparticles).

4. The method of claim 1, wherein the diselenide block copolymer is used for coating radiated nanoparticles and chemotherapy drugs comprises: dissolving diselenide block copolymer and DSPE-PEG-biomarker into ultrapure water; adding dichloromethane organic solvent having oil phase radiated nanoparticles and doxorubicine dissolved, wherein the proportion of the diselenide block copolymer, the DSPE-PEG-biomarker, the oil phase radiated nanoparticles, and the doxorubicin is 5:1:1:1; completing emulsification with sonication in ice bath; and heating to 60°C to remove the dichloromethane organic solvent to obtain radiation-sensitive nanoparticles carrying the radiated nanoparticles and the doxorubicin (RNPs/DOX-Radiation-Sensitive nanoparticles).

5. The method of claim 2, wherein the RNPs-Radiation-Sensitive nanoparticle is in size of about 100 nanometers, and the radiated nanoparticle is uranium-238.

6. The method of claim 3, wherein the DOX-Radiation-Sensitive nanoparticle is in size of about 100 nanometers, and the pharmaceutical is doxorubicin.

7. The method of claim 4, wherein the RNPs/DOX-Radiation-Sensitive nanoparticle is in size of about 120 nanometers, the radiated nanoparticle is uranium-238, and the pharmaceutical is doxorubicin.

## Patentansprüche

1. Verfahren zur Herstellung eines strahlungsempfindlichen Copolymerträgers zur Beschichtung von bestrahlten Nanopartikeln und / oder Chemotherapeutika, umfassend:
Lösen von festem Natriumhydroxid in Wasser und Zugabe von Selenpulver und Cetyltrimethylammoniumbromid, um eine Selenlösung zu erhalten;
Lösen von Natriumborhydrid und einem anderen festen Natriumhydroxid in Wasser in einem Eisbad, um eine Natriumborhydridlösung zu erhalten, und Instillieren der Selenlösung in die Natriumborhydridlösung zur Reaktion unter Raumtemperatur für etwa eine Stunde und anschließender Reaktion bei etwa 90°C für etwa eine halbe Stunde, um die Reaktion abzuschließen und eine alkalische Lösung aus Natriumselenid zu erhalten;
Auflösen von 2-Dodecen-1-yl-Bernsteinsäureanhydrid in Tetrahydrofuran und Zugabe in die alkalische Lösung von Natriumselenid zur Reaktion für etwa 12 Stunden, Entfernen von Verunreinigungen durch Säulenchromatographie unter Verwendung von wasserfreiem Na₂SO₄ oder MgSO₄, um Wasser zu entfernen, um Diselenid nach einer Hochtemperaturtrocknung zu erhalten ;
Auflösen des Diselenids in Tetrahydrofuran mit einem Polyethylenglycolpolymer mit einer Aminogruppe und Zugabe von 1-Ethyl-3 (-3-dimethylaminopropyl)carbodiimid oder N-Hydroxysuccinimid für eine etwa 12 Stunden lange Reaktion, um Verunreinigungen durch Säulenchromatographie zu entfernen, um ein Diselenid-Blockcopolymer nach einer Hochtemperaturtrocknung zu erhalten.

2. Verfahren nach Anspruch 1, wobei das Diselenid-Blockcopolymer zum Beschichten von bestrahlten Nanopartikeln verwendet wird, umfassend: Auflösen von Diselenid-Blockcopolymer und DSPE-PEG-Biomarker mit ultrareinem Wasser; Zugabe von organischem Dichlormethan-Lösungsmittel mit gelösten bestrahlten Ölphasen-Nanopartikeln, wobei der Anteil des Diselenid-Blockcopolymers, des DSPE-PEG-Biomarkers und der bestrahlten Ölphasen Nanopartikel 5: 1: 2 beträgt; Vervollständigung der Emulgierung mit Beschallung im Eisbad; Und Erhitzen auf 60°C, um das organische Dichlormethan-Lösungsmittel zu entfernen, um strahlungsempfindliche Nanopartikel zu erhalten, die die bestrahlten Nanopartikel (RNPs - strahlungsempfindliche Nanopartikel) transportieren.

3. Verfahren nach Anspruch 1, wobei das Diselenid-Blockcopolymer für die Beschichtung von Chemotherapie-Arzneimitteln verwendet wird, umfasst: das Auflösen von Diselenid-Blockcopolymer und DSPE-PEG-Biomarker mit Reinstwasser; Zugabe von organischem Dichlormethan Lösungsmittel mit gelöstem Doxorubicin, wobei der Anteil an Diselenid-Blockcopolymer, dem DSPE-PEG-Biomarker und dem Doxorubicin 5: 1: 2 beträgt; Vervollständigung der Emulgierung mit Beschallung im Eisbad; Und Erhitzen auf 60°C, um das organische Dichlormethan-Lösungsmittel zu entfernen, um strahlungsempfindliche Nanopartikel zu erhalten, die das Doxorubicin (DOX-strahlungsempfindliche Nanopartikel) transportieren.

4. Verfahren nach Anspruch 1, wobei das Diselenid-Blockcopolymer zum Beschichten von bestrahlten Nanopartikeln und Chemotherapie-Arzneimitteln verwendet wird, umfassend: Auflösen von Diselenid-Blockcopolymer und DSPE-PEG-Biomarker in ultrareines Wasser; Zugabe von organischem Dichlormethan Lösungsmittel mit Ölphasen bestrahlten Nanopartikeln und gelöstem Doxorubicin, wobei der Anteil des Diselenidblockcopolymers, des DSPE-PEG-Biomarkers, der mit Ölphase bestrahlten Nanopartikel und des Doxorubicins 5:1:1:1 beträgt; Vervollständigung der Emulgierung mit Beschallung im Eisbad; Und Erhitzen auf 60°C, um das organische Dichlormethan-Lösungsmittel zu entfernen, um strahlungsempfindliche Nanopartikel zu erhalten, die die bestrahlten Nanopartikel und das Doxorubicin (RNPs/ DOX-strahlungsempfindliche Nanopartikel) transportieren.

5. Verfahren nach Anspruch 2, wobei das RNPs-strahlungsempfindliche Nanopartikel eine Größe von etwa 100 Nanometern aufweist und das bestrahlte Nanopartikel Uran-238 ist.

6. Verfahren nach Anspruch 3, wobei das DOX-strahlungsempfindliche Nanopartikel eine Größe von etwa 100 Nanometern aufweist und das Pharmazeutikum Doxorubicin ist.

7. Verfahren nach Anspruch 4, wobei das RNPs/ DOX-strahlungsempfindliche Nanopartikel eine Größe von etwa 120 Nanometern aufweist, das bestrahlte Nanopartikel Uran-238 ist und das Pharmazeutikum Doxorubicin ist.

## Revendications

1. Procédé de fabrication d'un support copolymère sensible au rayonnement pour l'enrobage de nanoparticules irradiées et/ou de médicaments de chimiothérapie, comprenant :
la dissolution d'hydroxyde de sodium solide dans de l'eau et l'ajout de poudre de sélénium et de bromure de cétyltriméthylammonium afin d'obtenir une solution de sélénium ;
la dissolution de borohydrure de sodium et d'un autre hydroxyde de sodium solide dans de l'eau dans un bain de glace afin d'obtenir une solution de borohydrure de sodium, et l'instillation de la solution de sélénium dans la solution de borohydrure de sodium pour faire réagir à température ambiante pendant environ une heure, puis faire réagir à environ 90 °C pendant environ une demi-heure afin de terminer la réaction et d'obtenir une solution alcaline de séléniure de sodium ;
la dissolution d'anhydride 2-dodécén-1-ylsuccinique dans du tétrahydrofuranne et l'ajout dans la solution alcaline de séléniure de sodium pour faire réagir pendant 12 heures, la séparation d'impuretés par chromatographie sur colonne et l'utilisation de Na₂SO₄ ou de MgSO₄ anhydre pour supprimer l'eau afin d'obtenir du diséléniure après séchage à haute température ;
la dissolution du diséléniure dans du tétrahydrofuranne avec un polymère polyéthylène glycol comportant un groupe amino, et l'ajout de 1-éthyl-3-(3-diméthylaminopropyl) carbodiimide ou de N-hydroxysuccinimide pour faire réagir pendant environ 12 heures, la séparation d'impuretés par chromatographie sur colonne afin d'obtenir un copolymère à bloc de diséléniure après séchage à haute température.

2. Procédé selon la revendication 1, dans lequel l'utilisation du copolymère à bloc de diséléniure pour enrober des nanoparticules irradiées comprend : la dissolution du copolymère à bloc de diséléniure et de DSPE-PEG-biomarqueur avec de l'eau ultra-pure ; l'ajout de solvant organique chlorure de méthylène possédant des nanoparticules irradiées en phase huileuse dissoutes, la proportion du copolymère à bloc de diséléniure, du DSPE-PEG-biomarqueur, et des nanoparticules irradiées en phase huileuse étant de 5:1:2 ; l'achèvement de l'émulsification par sonication dans un bain de glace ; et le chauffage à 60 °C pour supprimer le solvant organique chlorure de méthylène afin d'obtenir des nanoparticules sensibles au rayonnement qui portent les nanoparticules irradiées (nanoparticules sensibles au rayonnement à RNP).

3. Procédé selon la revendication 1, dans lequel l'utilisation du copolymère à bloc de diséléniure pour enrober des médicaments de chimiothérapie comprend : la dissolution du copolymère à bloc de diséléniure et de DSPE-PEG-biomarqueur avec de l'eau ultra-pure ; l'ajout de solvant organique chlorure de méthylène possédant de la doxorubicine dissoute, la proportion du copolymère à bloc de diséléniure, du DSPE-PEG-biomarqueur, et de la doxorubicine étant de 5:1:2 ; l'achèvement de l'émulsification par sonication dans un bain de glace ; et le chauffage à 60 °C pour supprimer le solvant organique chlorure de méthylène afin d'obtenir des nanoparticules sensibles au rayonnement qui portent la doxorubicine (nanoparticules sensibles au rayonnement à DOX).

4. Procédé selon la revendication 1, dans lequel l'utilisation du copolymère à bloc de diséléniure pour enrober des nanoparticules irradiées et des médicaments de chimiothérapie comprend : la dissolution du copolymère à bloc de diséléniure et de DSPE-PEG-biomarqueur avec de l'eau ultra-pure ; l'ajout de solvant organique chlorure de méthylène possédant des nanoparticules irradiées en phase huileuse et de la doxorubicine dissoutes, la proportion du copolymère à bloc de diséléniure, du DSPE-PEG-biomarqueur, des nanoparticules irradiées en phase huileuse et de la doxorubicine étant de 5:1:1:1 ; l'achèvement de l'émulsification par sonication dans un bain de glace ; et le chauffage à 60 °C pour supprimer le solvant organique chlorure de méthylène afin d'obtenir des nanoparticules sensibles au rayonnement qui portent les nanoparticules irradiées et la doxorubicine (nanoparticules sensibles au rayonnement à RNP/DOX).

5. Procédé selon la revendication 2, dans lequel la nanoparticule sensible au rayonnement à RNP a une taille d'environ 100 nanomètres, et la nanoparticule irradiée est de l'uranium 238.

6. Procédé selon la revendication 3, dans lequel la nanoparticule sensible au rayonnement à DOX a une taille d'environ 100 nanomètres, et le médicament est de la doxorubicine.

7. Procédé selon la revendication 4, dans lequel la nanoparticule sensible au rayonnement à RNP/DOX a une taille d'environ 120 nanomètres, la nanoparticule irradiée est de l'uranium 238 et le médicament est de la doxorubicine.
